# EUROPEAN PATENT APPLICATION

(11) **EP 0 526 732 A2**
(43) Date of publication of application: **10.02.1993**
(21) Application number: 92110952.6
(22) Date of filing: 29.06.1992
(51) Int. Cl.: C07D 213/80, C07D 213/803, C07D 231/20, C07D 333/38

(54) **Processes for the preparation of heterocyclic thiocarbamates**

(30) Priority: 11.07.1991 US 728399; 11.05.1992 US 880770
(71) Applicant: E.I. DU PONT DE NEMOURS AND COMPANY, Wilmington Delaware 19898 (US)
(72) Inventor: Chiang, George C., Wilmington, Delaware 19803 (US)
(74) Representative: Hildyard, Edward Martin

(57) **Abstract**

The invention relates to processes for preparing intermediates for commercial sulfonylurea herbicides and to the intermediate products thereof.

## Description

The invention relates to processes for preparing intermediates for commercial sulfonylurea herbicides and to the intermediate products thereof.

The sulfonylurea herbicides are an extremely potent class of herbicides discovered relatively recently which generally consist of a sulfonylurea bridge, -S0₂NHCONH-, linking two aromatic or heteroaromatic rings. Such herbicides have become commercially important. There is therefore, a continuing need to discover new processes for their preparation that offer advantages that add to their commercial desirability.

Lissavetzky, J., Corral, C. and Valdbomillos, A. M., Synthesis, 1984, 2, 172 discloses the preparation of III, where R¹ is ethyl.

Japanese Kokai Patent No. Hei 2[1990]-172986 and DBP 1,020,641 disclose methods of preparing 3-hydroxy-2-thiophene carboxylic acid derivatives from the reaction of mercaptoacetic acid derivatives with a,;8-diha!ogenopropionic acid derivatives in the presence of a base.

There is a continual need for finding better processes for the preparation of sulfonylurea herbicides and their intermediates.

### SUMMARY OF THE INVENTION

The present invention is a process for preparing a heterocyclic thiocarbamate from simple acyclic starting materials by a two-step, one-pot process illustrated in Equation 1, 2 and 3 without the isolation or separation of intermediates. This invention also includes the Newman-Kwart rearrangement of certain pyridine thiocarbamates as illustrated in Equation 4. 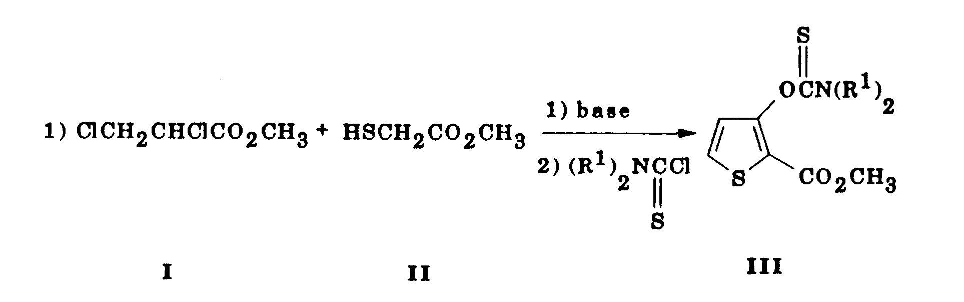 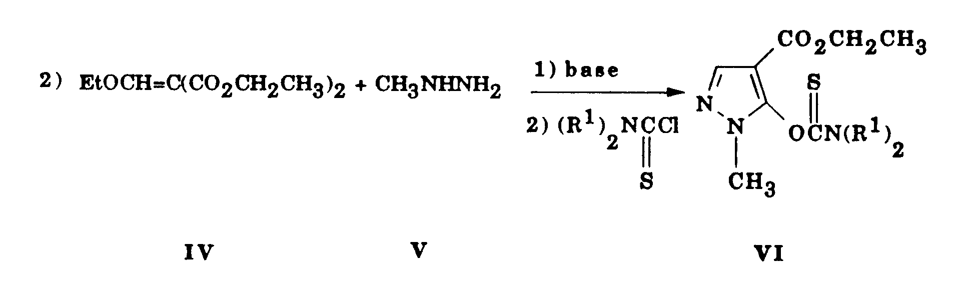 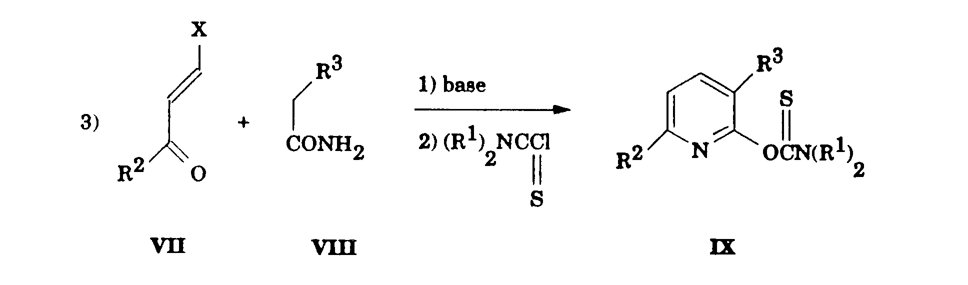 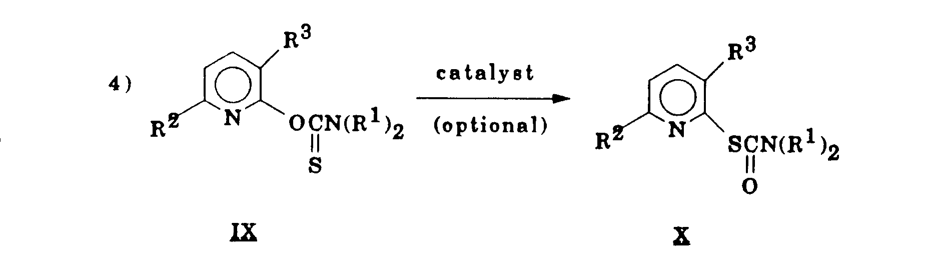
wherein:
R¹ is selected from C₁-C₂ alkyl;
R² is selected from hydrogen and CF₃;
R³ is selected from C0₂CH₃, C0₂CH₂CH₃, C(0)N(CH₃)₂, CF₃ and S0₂CH₂CH₃;
R⁴ is selected from C₁-C₄ alkyl;
R⁵ is selected from C₁-C₂ alkyl; and
X is selected from OR⁴ and N(R⁵)₂.

The reaction conditions for Equations 1-3 are generally as follows:
Solvents: Methanol, ethanol, propanol, isopropanol, acetonitrile and water.
Preferred solvents: Methanol and ethanol.
Temperatures: 0-100°C.
Preferred temperatures: 10-80 ° C.
Bases: NaOH, KOH, NaOMe, KOMe, KO-t-Bu, triethylamine and NaH.
Preferred bases: NaOH, KOH and NaOMe.
Time: 0.25-24 hours.
Preferred time: 2-12 hours.
Pressure: 1-5 atm.
Preferred pressure: 1 atm.

The reaction conditions for Equation 4 are generally as follows:
Solvents: Inert organic solvents such as toluene, benzene, chlorobenzene, dichlorobenzene, xylenes, methylene chloride, acetone, methyl ethyl ketone, acetonitrile, chloroform and tetrahydrofuran.
Preferred Solvents: Toluene, chlorobenzene, dichlorobenzene, methylene chloride, chloroform and xylenes.
Temperatures: 0-250 ° C.
Preferred Temperatures: 20-50 ° C.
Catalysts: Lewis acids such as BF₃, Lowry-Brønsted acids such as HCI and HBr, acylating agents such as acid chlorides and alkylating agents such alkyl or benzyl halides.
Preferred Catalysts: BF₃, HCI, HBr, acetyl chloride, methyl iodide and benzyl bromide.
Time: 0.25-96 hours.
Preferred time: 2-12 hours.
Pressure: 1-5 atm.
Preferred pressure: 1 atm.

Accordingly, the process of the invention is a two-step process carried out without separating intermediates, said process comprising first reacting in the presence of a solvent, a base, a temperature of 0-100°C and a pressure of 1-5 atmospheres 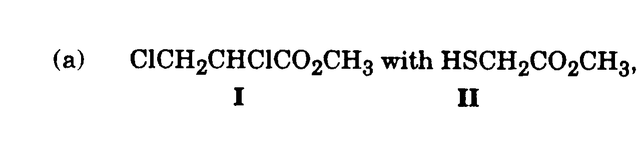 or 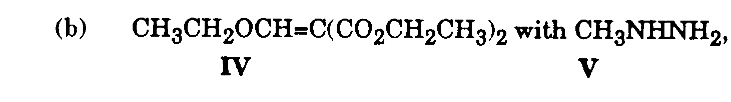 or 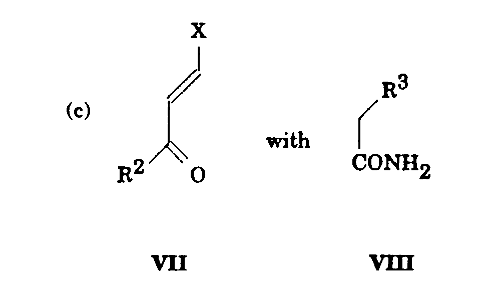
and then adding to the reaction mixture from (a), (b) or (c) after the reaction is complete (R¹)₂NC(=S)Cl to form in (a) 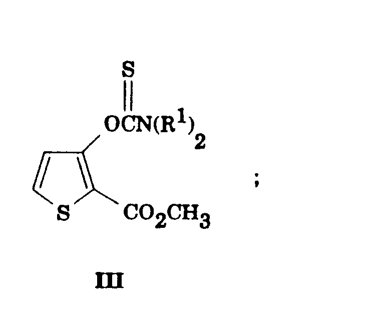
or in (b) 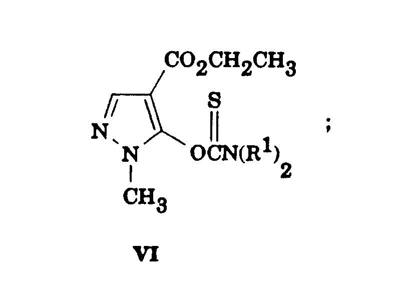
or in (c) 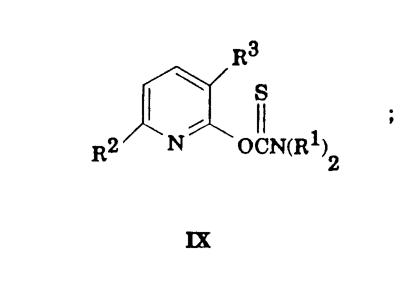
wherein:
R¹ is selected from C₁ -C₂ alkyl;
R² is selected from hydrogen and CF₃;
R³ is selected from C0₂CH₃, C0₂CH₂CH₃, C(0)N(CH₃)₂, CF₃ and S0₂CH₂CH₃;
R⁴ is selected from C₁ -C₄ alkyl;
R⁵ is selected from C₁-C₂ alkyl; and
X is selected from OR⁴ and N(R⁵)₂.

The second process of the invention is the further processing of compounds of Formula IX. Compounds of Formula IX readily undergo a Newman-Kwart rearrangement at low temperature, either neat or dissolved in a solvent, to the isomeric thiocarbamate of Formula X. The rearrangement can be accelerated with a variety of catalysts. 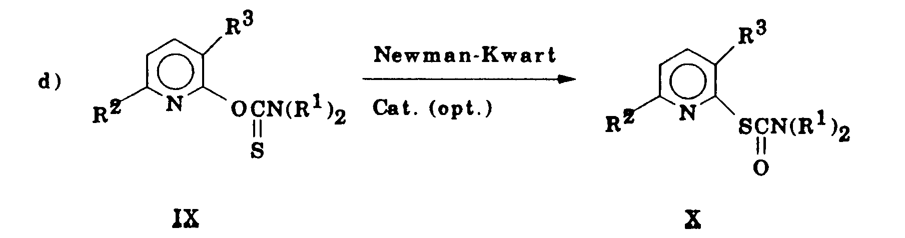
wherein:
R¹, R² and R³ are as previously defined.

Preferred processes of the present invention for reasons of ease of synthesis and efficiency are:
(1) The process of Equation 1 wherein the solvent is methanol and the base is selected from NaOH, KOH and NaOMe.
(2) The process of Equation 2 wherein the solvent is methanol and the base is selected from NaOH, KOH and NaOMe.
(3) The process of Equation 3 wherein the solvent is methanol and the base is selected from NaOH, KOH and NaOMe.
(4) The process of above Preferred 3 wherein R² is hydrogen and R³ is C(0)N(CH₃)₂.
(5) The process of above Preferred 3 wherein R² is hydrogen and R³ is SO₂CH₂CH₃.
(6) The process of Preferred 3 wherein R² is H and R³ is CF₃.
(7) The process of Preferred 3 wherein R² is CF₃ and R³ is C0₂CH₃.
(8) The process of Equation 4 wherein the solvent is toluene, chlorobenzene, dichlorobenzene or xylene.
(9) The process of Preferred 8 carried out in the presence of a catalyst selected from BF₃, HCI, HBr, acetyl chloride, methyl iodide and benzyl bromide.

The compounds of the invention are compounds of the Formula IX and X 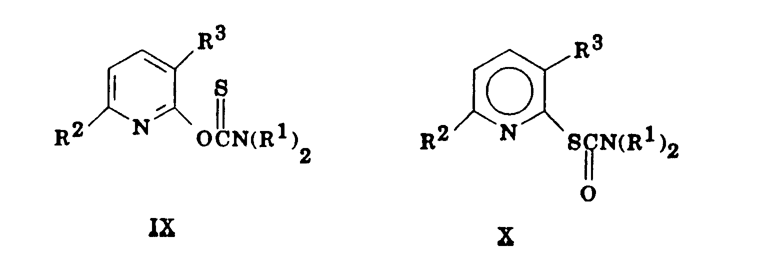
wherein:
R¹, R² and R³ are as defined above.

The compounds of the invention preferred for their efficiency and ease of synthesis are:
(10) The compounds of Formula IX wherein R² is H and R³ is C(0)N(CH₃)₂.
(11) The compounds of Formula IX wherein R² is H and R³ is S0₂CH₂CH₃.
(12) The compounds of Formula IX wherein R² is H and R³ is CF₃.
(13) The compounds of Formula IX wherein R² is CF₃ and R³ is C0₂CH₃.
(14) The compounds of Formula X wherein R² is H and R³ is C(0)N(CH₃)₂.
(15) The compounds of Formula X wherein R² is H and R³ is S0₂CH₂CH₃.
(16) The compounds of Formula X wherein R² is H and R³ is CF₃.
(17) The compounds of Formula X wherein R² is CF₃ and R³ is C0₂CH₃.

The process of Equations 1,2 and 3 of the invention can generally be carried out by reacting approximately equimolar amounts of the acyclic starting materials, that is, I and II or IV and V or VII and VIII in a solvent with 1 to 3.5 equivalents of base followed, after an appropriate reaction time, by the addition of dimethyl- or diethylthiocarbamoyl chloride. Typical reaction conditions are as follows. The preferred solvents are lower alcohols such as methanol, ethanol, propanol and isopropanol, other preferred solvents include acetonitrile and water. Methanol and ethanol are the most preferred solvents. Temperatures are 0 to 100°C. Preferred temperatures are 10 to 80°C. Preferred bases are sodium hydroxide, potassium hydroxide, sodium methoxide, potassium-t-butoxide and triethylamine. Most preferred bases are sodium hydroxide, potassium hydroxide and sodium methoxide. Reaction times are 0.25 to 24 hours, preferably 2 to 12 hours. Reaction pressures are 1 to 5 atm. Preferred pressure is 1 atm.

The process of Equation 4 of the invention can generally be carried out by dissolving compounds of Formula IX in an inert solvent such as o-dichlorobenzene, toluene or xylene and stirring. The rearrangement can be accelerated by warming the solution to 50-80 °C or by adding a catalyst selected from a Lewis acid, Lowry-Brønsted acid, acylating agents or alkylating agents. Preferred catalysts include protic acids such as HCI and HBr, simple acylating agents such as acetyl chloride, and simple alkylating agents such as methyl iodide and benzyl bromide. Reaction times are 0.25 to 96 hours, preferably 2 to 12 hours. Reaction pressures are 1 to 5 atm, preferably 1 atm.

The resulting products of the processes of the invention are useful as intermediates for the preparation of commercial herbicides such as methyl 3-[[[[(4-methoxy-6-methyl-1,3,5-triazinyl-2-yl)amino[carbonyl]-amino]sulfonyl]-2-thiophene carboxylate (Harmony@), 2-[[[[(4,6-dimethoxy-2-pyrimidinyl)amino]carbonyl]-amino]sulfonyl]-N,N-dimethyl-3-pyridine carboxamide (Accent@), N-[[(4,6-dimethoxy-pyridine-2-yl)amino]-carbonyl]-3-(ethylsulfonyl)-2-pyridinesulfonamide (Titus@) and N-[(4,6-dimethoxypyrimidin-2-yl)-aminocarbonyl]-1-methyl-4-ethoxycarbonyl-5-pyrazolesulfonamide.

### DETAILED DESCRIPTION OF THE INVENTION

The process of Equation 1 is conveniently carried out by reacting approximately equimolar amounts of I and II in a solvent such as methanol with approximately 3 equivalents of a base such as sodium methoxide. This reaction is exothermic and requires external cooling to maintain the reaction temperature below 35 ° C. After stirring for 1-2 hours, the reaction is completed and the methyl 3-hydroxy-2-thiophene carboxylate which is not isolated by an acid work-up, is converted directly to III by addition of N,N-dimethyl- or N,N-diethylthiocarbamoyl chloride. The product is conveniently isolated by diluting the reaction mixture with ice water and filtering off the crystalline-product III. N,N-dimethylthiocarbamoyl chloride is commercially available, while N,N-diethylthiocarbamoyl chloride can be prepared as described in Goshorn, R. H, Levis, W. W., Jaul, E., and Ritter, E. J., Org. Synthetic. Coll. Vol. IV, 307 (1963).

The process of Equation 2 is carried out by treating V, dissolved in an appropriate solvent, with 1.0-1.3 equivalents of a base, such as potassium hydroxide. The reaction mixture is then charged with an equimolar (relative to V) amount of IV. The addition of V is exothermic and may require external cooling. The reaction mixture is allowed to stir 1 to 3 hours followed by the addition of dimethyl- or diethylthiocarbamoyl chloride. After stirring, typically at room temperature, the product is conveniently isolated by filtration following dilution of the reaction mixture with ice water.

The process of Equation 3 is carried out by reacting of an approximately equimolar mixture of VII and VIII in an appropriate solvent, such as methanol, with 1-1.2 equivalents of base. The reaction mixture is stirred at ambient temperature for 0.5-2 hours and then refluxed for 2 to 18 hours. Upon cooling to ambient temperature, the reaction mixture is charged with dimethyl- or diethylthiocarbamoyl chloride, approximately one equivalent relative to VIII, and stirred at ambient temperature for 1-3 hours. The product is isolated by filtration after dilution of the mixture with ice water. Compounds of Formula VII, wherein R² is CF₃ and X is OR⁴, can be prepared as described in Hojo, M., Masuda, R., Kokuryo, Y., Shioda, H., and Matsuo, S., Chem. Lett., 499 (1976). Compounds of Formula VII, wherein R² is H, can be prepared by known methods from 1,3-propanedialdehyde. Compounds of Formula VIII are well-known and easily prepared by established procedures.

The compounds of Formula IX can also be prepared directly from the hydroxy-pyridine derivatives of Formula XI as illustrated in Equation 5. 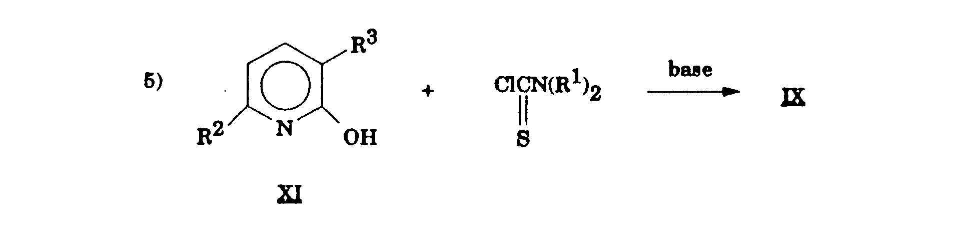 wherein:
R¹, R² and R³ are as previously defined.

The process of Equation 5 is conveniently carried out by treating a solution of XI dissolved in an organic solvent such as a lower alcohol, ether, tetrahydrofuran, dimethylformamide, methylene chloride or the like with a base selected from NaOH, KOH, NaH, NaOMe, triethylamine, pyridine or the like followed by treatment with dimethyl or diethylthiocarbamoyl chloride. The product of Formula IX can be isolated by a variety of conventional methods.

As an alternate to the ice-water dilution method and subsequent filtration as a convenient product isolation procedure for the processes of Equations 1,2 and 3, the products may be taken up in a suitable solvent by extraction of the reaction mixture. Suitable solvent include methylene chloride, chloroform, o-dichlorobenzene, monochlorobenzene, toluene, xylenes and 1,2-dichloroethane.

The compounds of Formula XI can also be isolated from the reaction mixture as described in Equation 3. That is, before treatment with the thiocarbamoyl chloride, the reaction mixture is acidified and XI isolated by conventional methods. The compounds of Formula XI are generally known in the literature or easily prepared by modifications of known procedures.

The process of Equation 4 is carried out by dissolving a compound IX in an appropriate solvent such as methylene chloride, dichlorobenzene or chloroform. The conversion to a compound of Formula X can be effected by allowing the solution to stand, heating the solution or adding an appropriate catalyst such as BF₃ or acetyl chloride. Heating the solution or the addition of catalyst greatly accelerates the rearrangement.

On standing, a solution of a compound of Formula IX will show the corresponding rearranged compound of Formula X. However, the conversion is slow, taking days even months to proceed to completion. Gently warming of the solution to approximately 50 ° C accelerates the rearrangement, with complete conversion 2-24 hours. Higher temperatures can be used as is done in conventional Newman-Kwart rearrangements, that is, temperatures from 150-250 °C, however, this is not economically justified. Finally, the rearrangement may be catalyzed by a variety of catalysts, most notably protic acids, Lewis acids such as BF₃ and acylating agents such as acetyl chloride. The use of a catalyst greatly accelerates the rearrangement, with complete conversion in usually one hour or less. The catalyst can range from 0.01 to 60 mol percent. Greater amounts of catalyst can be used, however, it is not economically justified.

The thiocarbamates formed by the process of this invention are useful intermediates for the manufacture of herbicides such as sulfonylureas.

The following Examples further illustrate the invention.

### EXAMPLE 1

### Synthesis of methyl 3-[(dimethylamino)thioxomethoxy]-2-thiophene carboxylate

In a 500 mL round-bottom flask equipped with a mechanical stirrer, a dropping funnel, a reflux condenser, and a thermometer was charged 75 mL, of methanol, 54.3 g of methyl 2,3-dichloropropionate and 36.5 g of methyl thioglycolate. The reactor was cooled in an ice water bath. Through the dropping funnel, 223 g, 1.03 mmol of 25% sodium methoxide was added to the reaction mixture. The reaction temperature was kept below 35 C, stirred at 25 °C for an hour and then cooled to 10 °C to allow the charge of 51.3 g N,N-dimethylthiocarbamoyl chloride. The mixture was stirred at 25 °C for another hour and then poured into 1000 mL ice water to crystallize the product. The crystals were filtered and washed with water. After drying overnight, the yield was 82% (68.2 g) of pure methyl 3-[(dimethylamino)thioxomethoxy]-2-thiophene-carboxylate, mp 92-94 °C.

NMR(DMSO-d₆): 6 7.90 (d, 1 H), 7.03 (d, 1 H), 3.78 (s, 3H), 3.35 (d, 6H). M/e: 245

### EXAMPLE 2

### Synthesis of methyl 3-[(dimethylamino)thioxomethoxy]-2-thiophene carboxylate

To a 500 mL flask equipped with a mechanical stirrer, a reflux condenser and a thermometer was charged 26.5 g of methyl thioglycolate, 39.3 g of methyl 2,3-dichloropropionate and 50 mL of methanol. It was cooled to 10°C and then dropwise added to a solution of 32 g of NaOH in 250 mLl methanol over half an hour. The temperature was kept below 35 °C during the addition. It was stirred for one hour at 25 °C and then cooled to 10°C to charge 32 g of N,N-dimethylthiocarbamoyl chloride. After stirring at 25-30 °C for 30 min, the pH of the reaction was 5. To keep the pH greater than 7, 10 g of NaOH pellets were charged. The reaction was stirred overnight and poured onto 500 mL ice water. The resulting crystals were filtered, washed with water and dried to yield 19.5 g (32%) of the compound of Example 1, mp 95-96 °C.

### EXAMPLE 3

### Synthesis of ethyl 5-[(dimethylamino)thioxomethoxy]-1-methyl-1 H-pyrazole-4-carboxylate

To a 50 mL round bottom flask equipped with a mechanical stirrer, a reflux condenser, a thermometer and a dropping funnel was charged 200 mL methanol, 13.3 g KOH pellets and 9.2 g of methylhydrazine. It was cooled to 10°C in an ice bath. Diethyl ethoxymethylenemalonate (43.2 g) was dropped into the reaction mixture and exotherm was observed. The mixture was stirred at 25 °C for 2 hours. N,N-dimethylthiocar- bamoyl chloride (25 g) was charged in one portion and the mixture stirred for another hour at 25 °C. The reaction mixture was poured into 300 mL ice water and the title compound crystallized. It was filtered and washed with water. The white crystals were dried overnight and weighed 35 g. The yield was 68% of ethyl 5-[(dimethylamino)thioxo-methoxy]-1-methyl-1 H-pyrazole-4-carboxylate, mp 119-120 ° C.

NMR (CDCl₃): 6 7.84 (s, 1 H), 4.25 (q, 2H), 3.90 (s, 3H), 3.40 (d, 6H), 1.30 (t, 3H). M/e: 257.

### EXAMPLE 4

### Synthesis of methyl 2-[(dimethylamino)thioxomethoxyl-6-trifluoromethyl-3-pyridine carboxylate

To a 1-I round bottom flask equipped with a mechanical stirrer, a reflux condenser, a thermometer and a dropping funnel was charged 44g of 4-butoxyl-1,1,1-trifluoro-3-buten-2-one, 29.5 g of ethyl malonate monoamide, and 300 mL methanol. Using a cooling bath, 60 g of 25% NaOMe solution was charged and a small exotherm was observed. Within half an hour, a brilliant yellow solid precipitated. The reaction mixture was heated to reflux overnight. Upon cooling to ambient temperature, 28 g of N,N-dimethylthiocarbamoyl chloride was charged and the reaction mixture was stirred at room temperature for 2 hours. The pH of the reaction mixture should be basic. It was poured into 1-I ice water and the precipitated crystals were filtered. After washing with water and drying, 48 g (70% yield) of methyl 2-[(dimethylamino)thioxomethoxy]-6-trifluoromethyl-3-pyridinecarboxylate, mp 94-95 °C was obtained.

NMR (CDCl₃): 6 3.45 (s, 6H), 3.90 (s, 3H), 7.70 (d, 1 H), 8.52 (d, 1 H).

### EXAMPLE 5

### Synthesis of methyl 2-[[(dimethylamino)carbonyl]thio]-3-pyridine carboxylate

In a 100 mL flask was charged 3 g KOH pellets, 60 mL methanol and 7 g methyl 2-hydroxynicotinate and the mixture was stirred at room temperature for one half hour. Afterwards, 5.7 g of N,N-dimethylthiocar- bamoyl chloride was added and the reaction mixture stirred for 4 hours. It was poured onto 100 mL ice water and filtered. The crystals were washed with water and dried to yield 5.3 g of methyl 2-[-(dimethylamino)thioxomethoxy]-3-pyridine carboxylate (48%).

NMR ((CH₃)₂SO-d₆): 6 3.40 (s, 6H), 3.80 (s, 3H), 7.50 (t, 1 H), 8.40(d, 1 H), 8.60 (d, 1 H), m.p. 125-127° C.

A solution of this compound (0.5 g) in 5 mL of CDCl₃ was treated with 0.16 g of BF₃ etherate at room temperature. Within one hour, NMR analysis (CDCl₃) indicated the conversion to the title compound was complete.

NMR (CDCl₃): 6 3.15 (br d, 6H), 3.95 (s, 3H), 7.60 (dd, 1H), 8.50 (d, 1H), 8.85 (d, 1H). M/e: 240.

### EXAMPLE 6

### Synthesis of methyl 2-[[(dimethylamino)carbonyl]thio]-6-(trifluoromethyl)-3-pyridine carboxylate

In a 500 mL flask was charged 6.4 g of KOH pellets and 300 mL methanol. Upon complete dissolution of KOH in methanol, a clean, colorless solution was formed. It was cooled to 10°C and charged wth 20 g of methyl-2-hydroxy-6-(trifluoromethyl)-3-pyridine carboxylate and stirred for one half hour. At 10°C, 11.2 g of N,N-dimethylthiocarbamoyl chloride was added and the reaction mixture stirred for 1 hour and then poured onto 500 mL ice water. The crystals were filtered, washed with water and dried to give 17.7 g of methyl 2-[-(dimethylamino)thioxomethoxy]-6-(trifluoromethyl)-3-pyridine carboxylate (63% yield).

NMR (CDCl₃): 6 3.48 (s, 6H), 3.90 (s, 3H), 7.30 (d, 1 H), 8.55 (d, 1 H). M/e: 308, m.p. 100-101 ° C.

A solution of this compound (0.5 g) dissolved in 5 mL of methylene chloride was treated with 0.16 g of BF₃ etherate at room tempterature. After one hour, the solvent was evaporated. NMR analysis indicated complete conversion of the title compound.

NMR (CDCl₃): δ 3.1 (br d, 6H), 3.90 (s, 3H), 7.70 (d, 1 H), 8.40 (d, 1 H). M/e: 308.

## Claims

1. A two-step process carried out without isolating intermediates, said process comprising first reacting in the presence of a solvent, a base and at a temperature of 0-100 °C and a pressure of 1-5 atmospheres 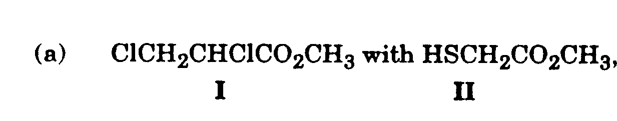
or 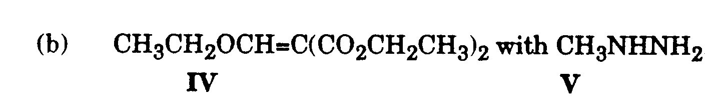
, or 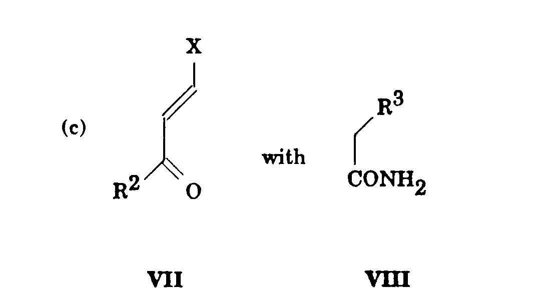
and then adding to the reaction mixture from (a), (b) or (c) after the reaction is complete (R¹)₂NC(=S)Cl to form in (a) 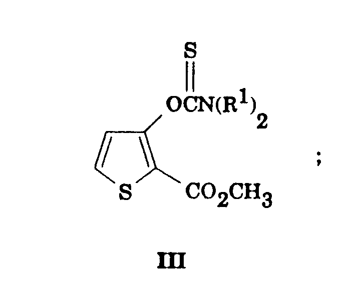
or in (b) 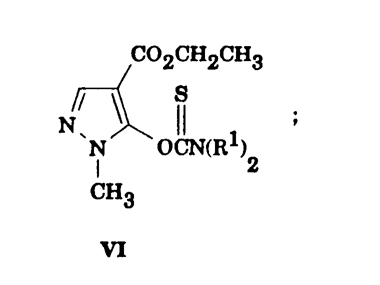
or in (c) 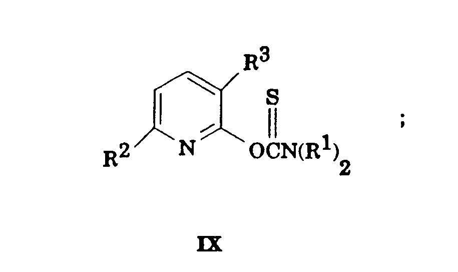
wherein:
R¹ is selected from C₁ -C₂ alkyl;
R² is selected from hydrogen and CF₃;
R³ is selected from C0₂CH₃, C0₂CH₂CH₃, C(0)N(CH₃)₂, CF₃ and S0₂CH₂CH₃;
R⁴ is selected from C₁ -C₄ alkyl;
R⁵ is selected from C₁-C₂ alkyl; and
X is selected from OR⁴ and N(R⁵)₂.

2. The process of Claim 1 wherein the solvent is selected from methanol, ethanol, propanol, isopropanol, acetonitrile and water and the base is selected from NaOH, KOH, NaOMe, KOMe, KO-t-Bu, triethylamine and NaH.

3. The process of Claim 2 wherein the reaction is (a), the solvent is methanol and the base is selected from NaOH, KOH and NaOMe.

4. The process of Claim 3 wherein the temperature is 10-80 °C and the pressure is 1 atmosphere.

5. The process of Claim 2 wherein the reaction is (b), the solvent is methanol and the base is selected from NaOH, KOH and NaOMe.

6. The process of Claim 5 wherein the temperature is 10-80 °C and the pressure is 1 atmosphere.

7. The process of Claim 2 wherein the reaction is (c), the solvent is methanol and the base is selected from NaOH, KOH and NaOMe.

8. The process of Claim 7 wherein the temperature is 10-80 °C and the pressure is 1 atmosphere.

9. The process of Claim 8 wherein R² is hydrogen and R³ is C(0)N(CH₃)₂.

10. The process of Claim 8 wherein R² is hydrogen and R³ is S0₂CH₂CH₃.

11. The process of Claim 8 wherein R² is hydrogen and R³ is CF₃.

12. The process of Claim 8 wherein R² is CF₃ and R³ is C0₂CH₃.

13. The process of Claim 1 wherein the compounds of Formula IX 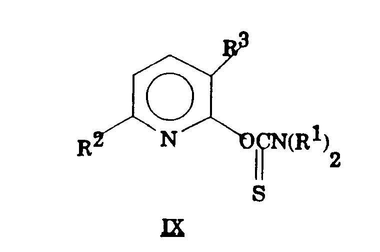
are reacted at a temperature of 20-50 °C and at a pressure of 1-5 atm in the presence of a catalyst and optionally an organic solvent for 0.25-96 hours to form X 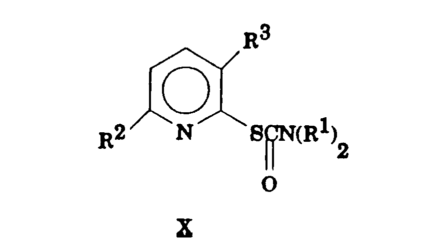
wherein:
R¹ is selected from C₁-C₂ alkyl;
R² is selected from hydrogen and CF₃; and
R³ is selected from C0₂CH₃, C0₂CH₂CH₃, C(0)N(CH₃)₂, CF₃ and S0₂CH₂CH₃.

14. The process of Claim 13 wherein a solvent is used.

15. The process of Claim 14 wherein the solvent is selected from toluene, chlorobenzene, dichlorobenzene and xylene.

16. The process of Claim 15 wherein the catalyst is selected from BF₃, HCI, HBr, acetyl chloride, methyliodide and benzyl bromide.

17. Compounds of Formula IX and X 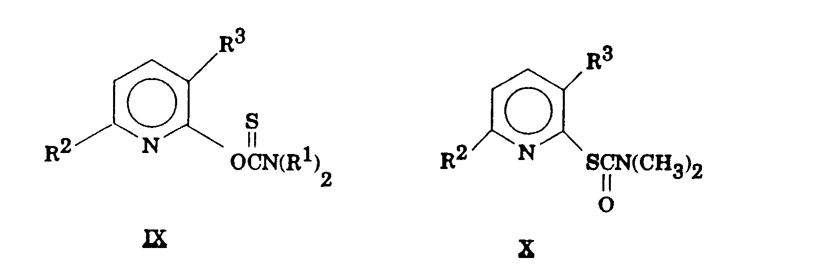
wherein:
R¹ is selected from C₁-C₂ alkyl;
R² is hydrogen or CF₃; and
R³ is selected from C0₂CH₃, C0₂CH₂CH₃, C(0)N(CH₃)₂, CF₃ and S0₂CH₂CH₃.

18. The compounds of Claim 17 wherein R² is hydrogen and R³ is S0₂CH₂CH₃.

19. The compounds of Claim 17 wherein R² is hydrogen and R³ is C(0)N(CH₃)₂.

20. The compounds of Claim 17 wherein R² is hydrogen and R³ is CF₃.

21. The compounds of Claim 17 wherein R² is CF₃ and R³ is C0₂CH₃.
